Europäisches Patentamt

European Patent Office

Office européen des brevets

(19)

(11) Numéro de publication: **0 229 092**

**B1**

(12) **FASCICULE DE BREVET EUROPEEN**

(45) Date de publication du fascicule du brevet: **29.08.90**

(21) Numéro de dépôt: **86903434.8**

(22) Date de dépôt: **18.06.86**

(86) Numéro de dépót international:
**PCT/FR86/00208**

(87) Numéro de publication internationale:
**WO 87/00057 15.01.87 Gazette 87/01**

(51) Int. Cl.⁵: **A 61 F 13/15,** A 61 F 13/52,
D 04 H 1/62

(54) INCORPORATION DE PRODUITS PULVERULENTS A L'INTERIEUR D'UN MATELAS DE FIBRES.

(30) Priorité: **24.06.85 FR 8509611**

(43) Date de publication de la demande:
**22.07.87 Bulletin 87/30**

(45) Mention de la délivrance du brevet:
**29.08.90 Bulletin 90/35**

(84) Etats contractants désignés:
**AT BE CH DE FR GB IT LI LU NL SE**

(56) Documents cités:
**EP-A-0 106 473**
**WO-A-80/01455**
**BE-A- 657 615**
**FR-A-2 305 944**
**FR-A-2 438 475**
**US-A-4 327 728**

(73) Titulaire: **KAYSERSBERG SA**
**Route de Lapoutroie**
**F-68240 Kaysersberg (FR)**

(72) Inventeur: **PIGNEUL, Raymond**
**2, rue des Vosges**
**F-68320 Durrenentzen (FR)**
Inventeur: **RUPPEL, Rémy**
**7, rue des Sorbiers**
**F-68000 Horbourg (FR)**

(74) Mandataire: **David, Daniel**
**KAYSERSBERG 54, avenue Hoche**
**F-75008 Paris (FR)**

## Description

L'invention concerne le domaine des articles à usage unique, destinés à absorber les liquides, comportant un matelas absorbant constitué de fibres, et vise un procédé et un dispositif permettant l'incorporation de produits pulvérulents, superabsorbants en particulier, à l'intérieur du matelas.

Afin de réduire le volume du matelas des articles à usage unique tels que couches pour bébé, changes pour incontinents, serviettes périodiques, protège-slips etc... ou bien augmenter leur capacité d'absorption, on cherche à incorporer au matelas des produits superabsorbants, appelés également additifs à rétention d'eau améliorée, hydrorétenteurs. Ces produits se présentent généralement sous la forme de granules, particules, poudres, ce sont des composés qui gonflent en présente de liquide mais qui sont insolubles; ils conduisent alors à la formation d'un gel dont le volume peut être de plusieurs dizaines de fois le volume du produit sec. Les produits les plus connus sont les alginates, les carboxyméthylcelluloses réticulés, les amidons greffés, les dérivés de synthèse du type acrylamide ou du type acrylate.

S'agissant de matériaux de nature différente: granules/fibres, leur mélange ne reste pas homogène dans le temps. Ce phénomène est d'autant plus marqué que les articles dont ils sont les composants subissent de nombreuses manipulations et déformations, pendant leur transport ou leur usage. On constate en effet une migration de la poudre vers des zones de l'article où elle devient inefficace.

La fixation des superabsorbants sur leur support a fait l'objet de nombreuses études. On peut les fixer par mouillage en assurant successivement leur gélification partielle, leur dépôt, puis leur séchage sur le support. Cette technique présente l'inconvénient de modifier l'état de surface des particules et par conséquent leur efficacité. Par ailleurs, l'étape de séchage que nécessite le procédé grève le coût de fabrication des articles.

On sait également assurer la fixation par des liants, comme cela est décrit dans les brevets US 3903889 et FR 2402474 où le produit est maintenu par une couche de matière adhésive. Cependant, il est apparu que le liant nuisait à l'expansion du produit et réduisait la surface active des granules.

Dans le brevet US 4055180 ou le brevet US 4381783 le superabsorbant est enfermé entre des feuilles supports liées entre elles de manière à ménager des poches. Ces feuilles peuvent être des feuilles imperméables percées d'ouvertures pour le passage des liquides ou être des feuilles perméables. On évite par ce moyen, la migration des particules pendant les manipulations de l'article. Mais on assure aussi le confinement du gel: on évite sa remontée vers le voile de couverture de l'article, côté corps, et on élimine tout risque de contact du gel avec la peau de l'utilisateur. Cependant, comme les parois de ces poches ne sont pas extensibles, l'action du produit en est limitée. Par ailleurs, ces feuilles supplémentaires forment obstacle au passage des liquides et nuisent à la rapidité de leur absorption.

On a cherché également à incorporer les particules directement dans le matelas absorbant. Le brevet US 3888257 décrit un procédé d'incorporation utilisant des jets d'air comprimé pour faire pénétrer les particules, préalablement déposées en surface, à l'intérieur du matelas de fibres. Ce procédé est de mise en oeuvre délicate, les jets d'air ayant tendance à déstabiliser le matelas et lui faire perdre son caractère homogène.

Le brevet FR 2446357 décrit un procédé où le produit pulvérulent est déposé entre un matelas et une nappe de fibres, liés entre eux selon des lignes ou des points, de manière à définir des espaces de confinement. Le liage est réalisé par simple matriçage à froid au moyen de cylindres gravés en motif à quadrillage ou par calandrage à chaud quand les fibres sont thermofusibles. L'avantage de cette solution est qu'elle peut être réalisée de façon très économique; elle assure un bon maintien des particules entre les nappes et elle met à profit l'élasticité des fibres pour permettre l'expansion du gel. Cependant dans cette solution le produit superabsorbant reste en surface du matelas et forme un dépôt continu.

Après un premier flux de liquide, le gel formé constitue un barrage uniforme limitant la diffusion ultérieure de liquide vers les couches profondes du matelas qui seraient susceptibles de l'absorber. Ainsi dans les changes, pour bébés par exemple, même en prévoyant une capacité d'absorption largement suffisante pour un usage normal, il se produit des fuites car les liquides ne sont pas absorbés suffisamment rapidement, suite à la formation du gel.

Pour résoudre ce problème, l'invention se propose de fragmenter le dépôt de poudre de manière à retarder la formation d'une barrière continue et permettre le passage des liquides vers les couches inférieures du matelas, et de localiser la poudre vers le fond du matelas de façon que les fibres, jouant le rôle de diffuseur des liquides vers le produit absorbant soient en majorité interposées entre la source de liquide et le superabsorbant.

Le procédé d'incorporation de produits pulvérulents notamment des produits superabsorbants à l'intérieur d'un matelas de fibres, telles que de la mousse de cellulose, pour un article à usage unique est caractérisé en ce qu'il consiste à comprimer localement le matelas au moyen d'un outil à profil déterminé, et à déposer le produit pulvérulent à l'intérieur de l'empreinte laissée par ledit outil.

L'empreinte formera de préférence un alvéole dont la section dépendra essentiellement de l'application, elle pourra être circulaire, oblongue, carrée, rectangulaire.

Le procédé concerne en particulier la fabrication d'articles à usage unique: couches, changes pour bébé ou adultes incontinents, serviettes périodiques, protège-slips, etc... dont le matelas de fibres est constitué de mousse de cellulose obtenue par défibrage à sec de feuilles de pâte à papier. Mais il concerne également les matelas contenant d'autres types de fibres, liées ou non liées.

Les produits pulvérulents visés sont essentiellement des superabsorbants mais d'autres additifs peuvent être incorporés de la même manière. Les superabsorbants susceptibles d'être utilisés sont les produits que l'on trouve dans le commerce sous forme pulvérulente: particules, granulés. Cette forme offre un rapport surface/volume optimum permettant une absorption maximale des molécules de liquides.

Conformément à une autre caractéristique de l'invention, aprés avoir déposé le produit dans les empreintes ou alvéoles on lui applique une force de compression vers le fond de l'alvéole de maniére à le sertir au moins partiellement dans la masse de fibres.

Conformément à une autre caractéristique de l'invention on répartit le produit dans une pluralité d'alvéoles disposés dans le matelas selon un motif déterminé.

Grâce à ce procédé on obtient un produit dont on maîtrise parfaitement, de façon simple, la répartition de la poudre dans la masse de maniére à optimiser son action.

On connaît déjà par le brevet US 4435178, un article d'hygiène dont le matelas absorbant comporte deux nappes superposées. Sur la nappe disposée côté corps, sont ménagés des alvéoles cylindriques ouverts vers la face de réception du liquide. Ces alvéoles ont pour fonction de canaliser les fluides vers l'intérieur du matelas et sont maintenus ouverts grâce à une enduction des parois avec un produit de consolidation pouvant accessoirement être un superabsorbant. La conception d'un tel article, relativement à l'enduction des alvéoles, ne semble pas réalisable économiquement selon un procédé simple permettant des cadences de production élevées. Par ailleurs la forme continue, non divisée, du superabsorbant fait douter de son efficacité.

Conformément à un autre aspect de l'invention le matelas peut être ensuite recouvert d'une nappe fibreuse, avec interposition d'une couche de liant, de manière à améliorer le confinement et limiter la remontée du gel. Avantageusement cette nappe de fibres est constituée de fibres cellulosiques papetiéres liées au moyen d'un latex selon la technique décrite par exemple dans le brevet FR 2418829.

La demande de brevet EP 106473 décrit un protège-slip, et un dispositif pour former des empreintes en surface, comprenant un cylindre muni de protubérances. Conformément à un autre aspect de l'invention, le dispositif de mise en oeuvre du procédé comprend un premier cylindre avec une pluralité de dents disposées en secteurs de cercles et un deuxième cylindre pourvu de dents disposé parallèlement au premier cylindre, en aval par rapport à la direction de déplacement du tapis, et entraîné en rotation en synchronisme avec le premier cylindre de manière que les dents du deuxième cylindre pénètrent dans les empreintes laissées par les dents du premier cylindre, le distributeur de poudre étant disposé entre les deux cylindres.

La description qui suit, en référence aux dessins annexés, se rapporte au mode de réalisation, à titre d'exemple non limitatif, d'un dispositif de mise en oeuvre du procédé:

La figure 1 représente en perspective isométrique le dispositif selon l'invention placé sur un matelas de fibres.

La figure 2 est une vue en coupe selon II-II de la figure 1.

La figure 3 est une vue en coupe transversale, à une échelle différente, d'un article d'hygiène à usage unique comportant des particules de superabsorbant incorporées dans le matelas de fibres selon le procédé de l'invention.

La figure 4 est une vue en coupe selon IV-IV de la figure 3.

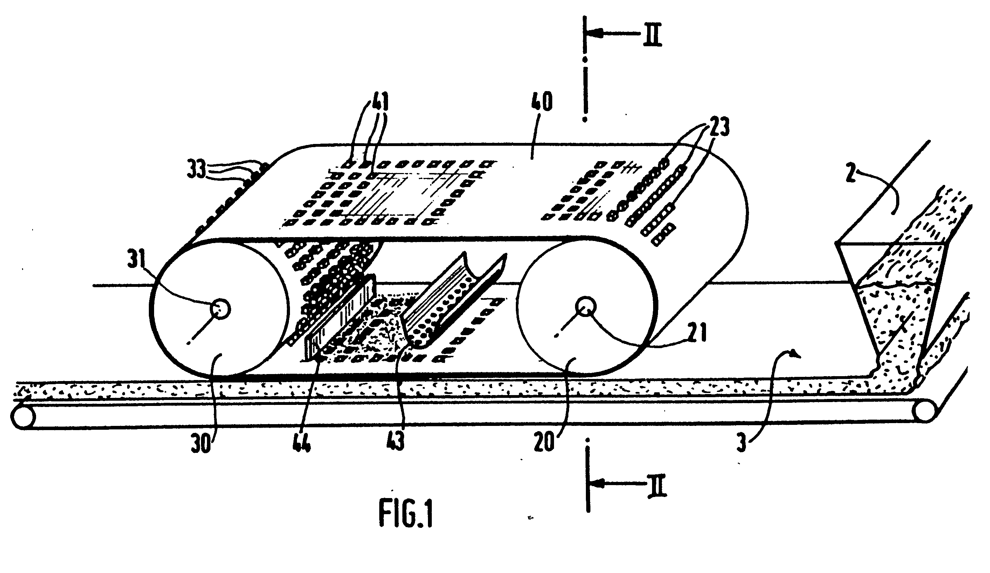

Sur la figure 1 est représenté schématiquement un dispositif d'insertion de superabsorbant, selon l'invention, intégré dans une ligne de fabrication en continu d'articles d'hygiène à usage unique dont n'est représentée que la partie amont de formation du matelas en mousse de cellulose. Celle-ci, après l'opération de défibrage à sec des feuilles de pâte à papier est introduite dans une trémie 2 par laquelle elle est distribuée sur un tapis transporteur pour former un matelas 3 de grammage et de dimensions déterminés.

Le dispositif selon l'invention est constitué de deux cylindres identiques 20, 30 montés rotatifs autour de deux axes parallèles 21, 31 et réunis par une courroie sans fin 40 sensiblement de même largeur que les cylindres. Le dispositif est suspendu au-dessus du matelas, les axes 21, 31 disposés transversalement par rapport à la direction de déplacement du matelas, et la partie inférieure de la courroie posée à plat sur le matelas sans exercer de force de compression.

La courroie et les cylindres sont dimensionnés et entraînés en synchronisme de manière que la courroie se déplace avec le matelas, à la même vitesse, sans glissement.

Les cylindres 20, 30 portent chacun des couronnes 22a, 22b, 22c... et 32a, 32b, 32c... identiques au moins deux par deux d'un cylindre à l'autre. Les couronnes d'un même cylindre sont parallèles les unes aux autres et disposées transversalement par rapport à l'axe du cylindre. Chaque couronne comporte un nombre déterminé de dents 23, 33 réparties en un ou plusieurs secteurs suivant l'application, deux en l'occurrence.

Chaque couronne 22a, 22b... du premier cylindre est identique et disposée dans le même plan que la couronne correspondante 32a, 32b,... du deuxième cylindre.

La courroie 40 comporte des perçages 41, disposés en pavés, correspondants pour un pavé, en nombre, dimensions et disposition aux dents d'un secteur de cylindre couvert par les dents. Cette disposition relative entre la courroie et les cylindres est de permettre selon une première fonction de la courroie, le parfait synchronisme entre les cylindres. Les dents de chaque couronne de chacun des cylindres s'engrènent dans les mêmes perçages que les dents correspondantes de la couronne correspondante de l'autre cylindre.

Les dents font saillie hors de la surface externe de la courroie. Leur hauteur est choisie en fonction de l'épaisseur du matelas. Les dents doivent pouvoir pénétrer dans le matelas pour le comprimer d'une profondeur déterminée sans le traverser pour autant. Les hauteurs sont de préférence égales d'un cylindre à l'autre.

Entre les deux cylindres et dans la boucle formée par la courroie est disposé un distributeur 43 de poudre de superabsorbant. Ce distributeur peut être constitué de façon connue d'une gouttière vibrante assurant le dépôt de la poudre sur une bande de largeur déterminée égale à la largeur des pavés formés par les perçages de la courroie. La poudre est donc déposée partie dans les perçages, partie sur la surface interne de la courroie entre les perçages. Une racle 44 disposée en aval du distributeur par rapport à la direction de translation de la courroie rabat dans les ouvertures de la courroie la poudre résiduelle. Eventuellement un dispositif de récupération non représenté élimine l'excès de poudre qui n'a pas été rapporté dans les ouvertures.

Ce dispositif fonctionne de la façon suivante:

Après sa formation, le matelas se déplace en translation sur le tapis transporteur jusqu'au niveau du premier cylindre 20 du dispositif, où les dents 23 d'un secteur impriment une série de rangées d'alvéoles dans la masse. En aval de ce cylindre, les dents se sont retirées laissant leur empreinte. Au droit du distributeur vibrant, une partie de la poudre déposée par la gouttière 43 sur la courroie, passe directement à travers les perçages 41 dans les alvéoles, une autre partie de la poudre est repoussée vers les alvéoles par le racle 44. Lorsque le matelas parvient au niveau du deuxième cylindre les dents 33 de ce dernier pénètrent à leur tour dans les alvéoles, formés par le premier cylindre, à travers les ouvertures 41 de la courroie et repoussent vers le fond les particules de produit superabsorbant qui s'y trouvent. Après rotation du cylindre les dents se retirent des alvéoles, le matelas poursuit sa translation incrusté de particules de superabsorbant déposées en quantité déterminée, au fond d'un nombre également déterminé d'alvéoles.

A ce matelas est ensuite associée une nappe de fibres liées par latex selon le procédé du brevet FR 2418829. Cette nappe est déposée sur le matelas après pulvérisation sur celui-ci de filets de colle thermofusible; le complexe ainsi formé est ensuite enveloppé entre un voile perméable en non-tissé constituant le côté de l'article venant contre la peau de l'utilisateur et une feuille imperméable, en polyéthylène par exemple, côté extérieur. Enfin, le matelas est scellé et découpé en articles individuels, la séparation étant pratiquée dans la zone ne comportant pas d'alvéoles entre deux desdits pavés.

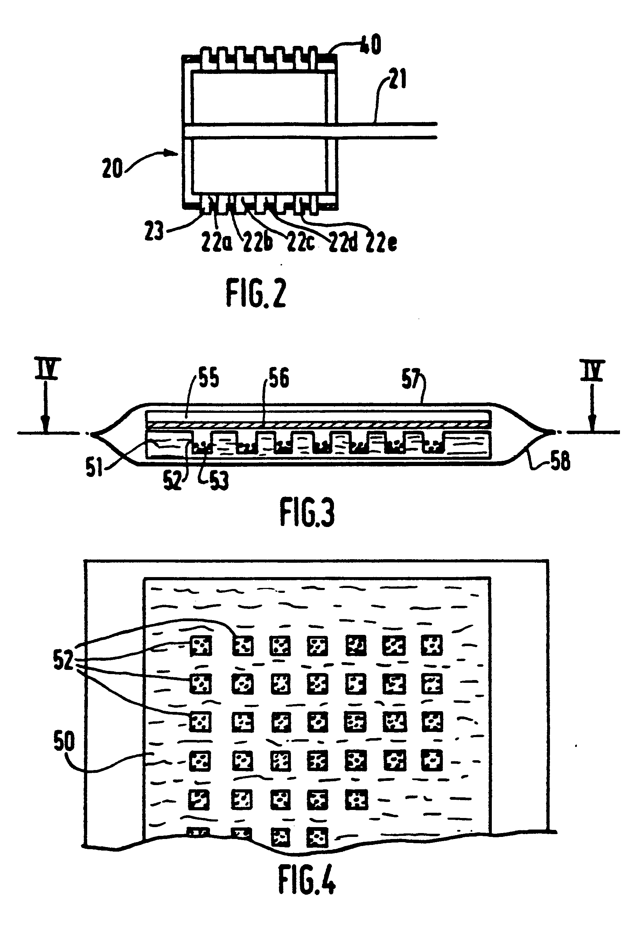

Les figures 3 et 4 représentent schématiquement la constitution d'un article ainsi obtenu dont les caractéristiques, données à titre d'exemple, peuvent être les suivantes.

Le matelas rectangulaire 51 est en mousse de cellulose de grammage 300 g/m², ses dimensions longitudinales et transversales sont de 430 mm et 145 mm, l'épaisseur moyenne est de 3,5 mm.

Le matelas comporte 24 rangées de 8 alvéoles 52, cylindriques, de 10 mm de diamètre et profonds de 1,7 mm. Le produit pulvérulent est localisé au fond de ces alvéoles à raison de 4,5 g par change, et est au moins partiellement incrusté dans la mousse de cellulose. Une nappe 55 de fibres papetières liées par un latex, de grammage 65 g/m², est associée au matelas par l'intermédiaire d'une couche 56 de matière adhésive déposée par enduction ou bien par la technique dite de pulvérisation de colle thermofusible. L'ensemble est enveloppé entre un voile perméable non-tissé 57 et une feuille 58 de polyéthylène imperméable, soudés bords à bords.

Grâce à cette localisation du superabsorbant en des zones distinctes, on ménage des zones de diffusion facilitant l'absorption des liquides. D'autre part, on retarde de cette façon la formation d'une couche continue de gel.

## Revendications

1. Procédé d'incorporation d'un produit pulvérulent, notamment superabsorbant à l'intérieur d'un matelas de fibres, en particulier de mousse de cellulose, pour articles à usage unique, caractérisé en ce qu'il consiste à comprimer localement ledit matelas au moyen d'un outil à profil déterminé et à déposer le produit pulvérulent à l'intérieur de l'empreinte laissée par l'outil.

2. Procédé selon la revendication 1, caractérisé en ce que l'empreinte forme un alvéole.

3. Procédé selon la revendication 2, caractérisé en ce qu'après son dépôt, on applique au produit une force de compression dirigée vers le fond de l'alvéole.

4. Procédé selon l'une des revendications précédentes, caractérisé en ce qu'on réalise une pluralité d'empreintes disposées selon un motif déterminé.

5. Procédé selon l'une des revendications précédentes, caractérisé en ce qu'on associe au matelas une nappe de fibres supplémentaire de manière à recouvrir les empreintes.

6. Procédé selon la revendication 5, caractérisé en ce qu'on lie la nappe de fibres au matelas au moyen d'une substance adhésive.

7. Procédé selon l'une des revendications 5 ou 6, caractérisé en ce que la nappe supplémentaire est une nappe de fibres liées, notamment par latex.

8. Dispositif pour la mise en oeuvre du procédé selon la revendication 4 dans une ligne de fabrication d'articles à usage unique où le matelas de fibres est déposé en continu sur un tapis transporteur, caractérisé en ce qu'il comprend un premier cylindre (20) monté rotatif autour d'un axe transversal à la direction de déplacement du tapis comportant une pluralité de dents (23) disposées en secteurs de cercles et un deuxième cylindre (30) pourvu de dents (33), disposé parallèlement au premier cylindre, en aval par rapport à la direction de déplacement du tapis, et entraîné en rotation en synchronisme avec le premier cylindre de manière que les dents (33) du deuxième cylindre pénètrent dans les empreintes laissées par les dents (23) du premier cylindre, le distributeur (43) de poudre étant disposé entre les deux cylindres.

9. Dispositif selon la revendication 8, caractérisé en ce qu'une courroie sans fin relie les deux cylindres et comporte des perçages (41) lui permettant de s'engréner sur les dents (23, 33) des cylindres.

10. Dispositif selon la revendication 9, caractérisé en ce que les perçages (41) sont disposés en pavés correspondant à la disposition en secteur des dents des cylindres.

11. Dispositif selon la revendication 10, caractérisé en ce que le distibuteur (43) est placé à l'intérieur de la boucle formée par la courroie.

12. Dispositif selon la revendication 11, caractérisé en ce qu'un racle (44) est disposé entre le distributeur et le deuxiéme cylindre.

## Patentansprüche

1. Verfahren zur Einarbeitung eines pulverförmigen Produktes, insbesondere eines superabsorbierenden Produktes, in das Innere eines Faserkissens, insbesondere aus Zelluloseschaum, von Einmalartikeln, dadurch gekennzeichnet, daß das Kissen lokal mit Hilfe eines Werkzeuges von bestimmtem Profil eingedrückt wird und daß das pulverförmige Produkt in das Innere der von dem Werkzeug hergestellten Abdrücke deponiert wird.

2. Verfahren nach Anspruch 1, dadurch gekennzeichnet, daß der Abdruck eine Wabe formt.

3. Verfahren nach Anspruch 2, dadurch gekennzeichnet, daß nach seiner Deponierung auf das Produkt eine Kompressionskraft ausgeübt wird, die zum Boden der Wabe gerichtet ist.

4. Verfahren nach einem der vorhergehenden Ansprüche, dadurch gekennzeichnet, daß eine Reihe von Abdrücken realisiert wird, die entsprechend eines bestimmten Motives angeordnet sind.

5. Verfahren nach einem der vorhergehenden Ansprüche, dadurch gekennzeichnet, daß das Kissen mit einem zusätzlichen Faservlies zusammengebracht wird, derart, daß die Abdrücke bedeckt sind.

6. Verfahren nach Anspruch 5, dadurch gekennzeichnet, daß der Faservlies mit dem Kissen über eine klebende Substanz verbunden wird.

7. Verfahren nach einem der Ansprüche 5 oder 6, dadurch gekennzeichnet, daß der zusätzliche Vlies ein Vlies aus Fasern ist, die insbesondere mit Latex untereinander verbunden sind.

8. Vorrichtung zur Durchführung des Verfahrens nach Anspruch 4 in einer Fabrikationsstraße zur Herstellung von Einmalartikeln, wo das Faserkissen ununterbrochen auf einem Förderband deponiert wird, dadurch gekennzeichnet, daß sie einen ersten Zylinder (20) enthält, der drehbar um eine transversal zu der Förderrichtung des Bandes ausgerichteten Achse angeordnet ist und eine Mehrzahl von Zähnen (23) enthält, die kreissektorförmig angeordnet sind und daß ein zweiter mit Zähnen (33) versehener Zylinder (30) vorgesehen ist, der parallel zum ersten Zylinder und flußabwärts im Verhältnis zu der Fortbewegungsrichtung des Förderbandes angeordnet ist und der in Synchrondrehung mit dem ersten Zylinder angetrieben wird derart, daß die Zähne (33) des zweiten Zylinders in die von den Zähnen (23) des ersten Zylinders erzeugten Abdrücke eindringen und wobei die Verteilervorrichtung (43) für das pulver zwischen den beiden Zylindern angeordnet.

9. Vorrichtung nach Anspruch 8, dadurch gekennzeichnet, daß die beiden Zylinder durch einen Endlosriemen verbunden sind, der Einstanzungen (41) aufweist, die es ihm erlauben, mit den Zähnen (23, 33) des Zylinders in Eingriff zu kommen.

10. Vorrichtung nach Anspruch 9, dadurch gekennzeichnet, daß die Einstanzungen (41) in Mustern, angeordnet sind, die der sektorweisen Anordnung der Zähne des Zylinders entsprechen.

11. Vorrichtung nach Anspruch 10, dadurch gekennzeichnet, daß die Verteilervorrichtung (43) im Inneren der durch den Riemen gebildeten Schleife angeordnet ist.

12. Vorrichtung nach Anspruch 11, dadurch gekennzeichnet, daß eine Schabeklinge (44) zwischen der Verteilervorrichtung und dem zweiten Zylinder angeordnet ist.

## Claims

1. A method of incorporating a powdered product, in particular a superabsorbent product, inside a pad composed of fibres, in particular of cellulose foam, for disposable articles, characterised in that it consists in compressing the pad locally by means of a tool with a specified profile, and in depositing the powdered product inside the imprint left by said tool.

2. A method according to claim 1, characterised in that the imprint forms a pocket.

3. A method according to claim 2, characterised in that after being deposited, the product is subjected to a compression force directed towards the bottom of the pocket.

4. A method according to one of the preceding claims, characterised in that a plurality of imprints are formed, arranged according to a specified pattern.

5. A method according to one of the preceding claims, characterised in that a supplementary fibre fabric is joined to the pad so as to cover the imprints.

6. A method according to claim 5, characterised in that the fibre fabric is joined to the pad by means of an adhesive substance.

7. A method according to one of claims 5 or 6, characterised in that the supplementary fabric is a fabric of fibres joined in particular with latex.

8. Apparatus for carrying out the method according to claim 4 in a production line for disposable articles, wherein the fibre pad is laid continuously on a conveyor belt, characterised in that it comprises a first cylinder (20), which is mounted rotatably about a shaft transverse to the direction of movement of the belt and which comprises a plurality of teeth (23) arranged in sectors of circles, and a second cylinder (30) provided with teeth (33), which is mounted parallel to the first cylinder, downstream in the direction of movement of the conveyor belt, and which is driven in rotation in synchronisation with the first cylinder, so that the teeth (33) of the second cylinder penetrate into the imprints left by the teeth (23) of the first cylinder, the powder distributor (43) being mounted between the two cylinders.

9. Apparatus according to claim 8, characterised in that the endless belt joins the two cylinders and comprises perforations (41) permitting the belt to mesh with the teeth (23, 33) of the cylinders.

10. Apparatus according to claim 9, characterised in that the perforations (41) are arranged in grids corresponding to the arrangement in a sector of the teeth of the cylinder.

11. Apparatus according to claim 10, characterised in that the distributor (43) is located inside the loop formed by the belt.

12. Apparatus according to claim 11, characterised in that a scraper (44) is mounted between the distributor and the second cylinder.

FIG.1

EP 0 229 092 B1

FIG.2

FIG.3

FIG.4